# EUROPÄISCHE PATENTSCHRIFT

(11) **EP 2 129 415 B1**
(45) Veröffentlichungstag und Bekanntmachung des Hinweises auf die Patenterteilung: **24.10.2018**
(21) Anmeldenummer: 08714768.2
(22) Anmeldetag: 19.03.2008
(51) Int. Cl.: A61M 5/20, A61M 5/32

(54) **INJEKTIONSVORRICHTUNG MIT GESTEUERTEM NADELRÜCKZUG**
INJECTION DEVICE WITH CONTROLLED NEEDLE RETRACTION
DISPOSITIF D'INJECTION POURVU D'UNE AIGUILLE DONT LE RETRAIT EST COMMANDÉ

(30) Priorität: 22.03.2007 DE 102007013836
(43) Veröffentlichungstag der Anmeldung: 09.12.2009
(73) Patentinhaber: Tecpharma Licensing AG, 3401 Burgdorf (CH)
(72) Erfinder: STREIT, Ursina, 3322 Schönbühl (CH); BOLLENBACH, Markus, CH-3014 Bern (CH); HOSTETTLER, Patrick, CH-3415 Hasle-Rüegsau (CH); KÜNZLI, Daniel, CH-4513 Langendorf (CH)
(86) Internationale Anmeldenummer: PCT/CH2008/000118
(87) Internationale Veröffentlichungsnummer: WO 2008/113198

(56) Entgegenhaltungen:
- EP-A- 0 516 473
- EP-A- 1 743 666
- WO-A-99/03529
- WO-A-2004/024211
- WO-A-2004/054645
- DE-U1-202007 000 578
- FR-A- 2 774 294

## Beschreibung

Die Erfindung liegt auf dem Gebiet der Injektionsvorrichtungen zum Verabreichen eines flüssigen Produkts, insbesondere Medikaments. Die Erfindung betrifft eine Injektionsvorrichtung zum gesteuerten Rückzug einer Nadel aus einer Einstechposition.

Bei Injektionsvorrichtungen, insbesondere Autoinjektoren ist es bekannt, eine Nadel durch den Mechanismus der Vorrichtung in einen Patienten zu injizieren und anschließend das Produkt durch die Nadel in den Patienten auszuschütten. Nach erfolgter Produktausschüttung wird die Nadel wieder zurück in die Injektionsvorrichtung gezogen.

Beispielsweise ist aus der US 6,387,078 B1 eine Injektionsvorrichtung bekannt, bei der eine Nadel mit Hilfe einer Vortriebsfeder zuerst eingestochen und dann mit dieser Vortriebsfeder die Produktausschüttung vorgenommen wird. Beim Vortrieb durch die Vortriebsfeder wird gleichzeitig eine Rückzugsfeder gespannt. Unmittelbar mit Erreichen der Endposition einer Ausschüttbewegung wird die Vortriebsfeder von der Rückzugsfeder entkoppelt, so dass die Rückzugsfeder die Nadel sofort in das Gehäuse zurückzieht. Hierbei kann es vorkommen, dass das Medikament nicht vollständig in das Gewebe des Patienten injiziert wird, so dass ein kleiner Rest verloren geht. Insbesondere bei teueren Medikamenten ist dies ärgerlich. Auch hinsichtlich der tatsächlich verabreichten Dosis kann der verloren gegangene Rest zu Problemen führen.

Aus der WO 9903529 ist ein Injektionsgerät bekannt mit einer Antriebsfeder und einer Rückzugsfeder, bei welchem ein Rückzug der Nadel in das Injektionsgerät durch die Rückzugsfeder erst durch Loslassen eines seitlichen Halteknopfs ermöglicht wird.

Die DE 20 2007 000 578 U1 offenbart eine Injektionsvorrichtung mit den Merkmalen des einleitenden Teils des Anspruchs 1.

Aufgabe ist es daher, eine Injektionsvorrichtung anzugeben, bei welcher der Patient auf einfache Weise bestimmen kann, wann die Nadel eingezogen wird.

Die Aufgabe wird gelöst durch die Merkmale des Anspruches 1. Vorteilhafte Weiterentwicklungen ergeben sich aus den abhängigen Ansprüchen.

Die Injektionsvorrichtung kann beispielsweise dazu angepasst sein, ein Produkt aus einem Produktbehältnis auszuschütten. Bevorzugt ist die Injektionsvorrichtung ein sog. Autoinjektor, bei dem eine Mechanik vorgesehen ist, die ein selbsttätiges Einstechen der Injektionsnadel und eine anschließende Produktausschüttung ermöglicht. Nach der Produktausschüttung soll die Nadel wieder zurück in die Injektionsvorrichtung gezogen werden.

Die Injektionsvorrichtung umfasst ein Gehäuse. Das Gehäuse weist ein distales und ein proximales Ende auf. Das distale Ende ist das Ende, an dem die Nadel angeordnet ist. Das proximale Ende ist das dem distalen Ende gegenüberliegende Ende.

Die Vorrichtung umfasst ferner eine Betätigungshülse, die relativ zu dem Gehäuse bewegbar gelagert ist. Die Betätigungshülse ist mit dem distalen Ende der Injektionsvorrichtung an eine Injektionsstelle des Patienten ansetzbar. Bevorzugt steht die Betätigungshülse über das distale Ende des Gehäuses ein kleines Stück über. Das kleine Stück soll so bemessen sein, dass bei der Bewegung der Betätigungshülse um dieses kleine Stück in proximale Richtung und zurück in die distale Richtung notwendige Schaltvorgänge ausgeführt werden können. Beispielsweise kann dies bei der Bewegung in proximale Richtung die Auslösung einer Einstechbewegung und bei der Bewegung zurück in distale Richtung die Auslösung der Rückzugsbewegung für die Nadel sein. Insbesondere kann ein Elastizitätsmittel, wie z.B. ein Federelement oder eine Feder vorgesehen sein, welches durch die Bewegung der Betätigungshülse in proximale Richtung spannbar ist und/oder mit dem die Betätigungshülse für die Bewegung in distale Richtung antreibbar ist. Das Elastizitätsmittel hat demnach einen rückstellenden Charakter und kann beispielsweise als Rückstellfeder bezeichnet werden. Bevorzugt dient das Elastizitätsmittel dazu, eine zur Auslösung eines Schaltvorgangs nicht ausreichend in die proximale Richtung zurück bewegte Betätigungshülse wieder in die Ausgangsposition zurückzustellen und/oder bei einem Schaltvorgang mitzuwirken, der bei der Bewegung der Betätigungshülse in distale Richtung verursacht wird.

Vorzugsweise kann die Betätigungshülse aus ihrer distalsten Position soweit in proximale Richtung geschoben werden, dass ihr distales Ende in etwa bündig mit dem distalen Ende des Gehäuses ist, was dem Zustand entspricht bei dem der Verwender die Injektionsvorrichtung an die Injektionsstelle des Patienten angesetzt und aufgedrückt hat.

Die Vorrichtung umfasst ferner eine Injektionsnadel, welche in einer Einstechposition über das distale Ende der Injektionsvorrichtung hervorsteht. Die Injektionsnadel kann z.B. an einem Produktbehältnis, insbesondere an dessen distalen Ende, angeordnet sein. Zumindest ist die Injektionsnadel fluidisch mit dem inneren des Produktbehältnisses verbunden. Im inneren des Produktbehältnisses befindet sich das zu verabreichende, insbesondere flüssige Produkt. Bei diesem Produkt kann es sich um ein Hormon, wie z.B. ein Wachstumshormon, oder um Insulin zur Diabetesbehandlung oder um ein anderes Medikament handeln. Das Produktbehältnis ist an seinem proximalen Ende mit einem relativ zum Produktbehältnis verschiebbar angeordneten Kolben verschlossen. Bei Bewegung des Kolbens in Richtung distalem Ende des Produktbehältnisses wird das Produkt über die Nadel ausgeschüttet. Bevorzugt ist die Nadel mit dem Produktbehältnis axial fest verbunden.

Bevorzugt sind die Injektionsnadel und insbesondere auch das Produktbehältnis relativ zum Gehäuse entlang der Längsachse der Injektionsvorrichtung verschiebbar angeordnet. Z.B. kann die Injektionsnadel von einer Ausgangsposition, in der sie sich insbesondere vollständig in dem distalen Ende der Injektionsvorrichtung befindet, d.h., nicht über das distale Ende der Vorrichtung hervorsteht, in die Einstechposition bewegt werden. In der Einstechposition steht die Nadel über das distale Ende der Vorrichtung um solch einen Weg über, der der Tiefe der Injektion der Nadel in das Gewebe des Patienten entspricht. Die Injektionsnadel ist aus der Einstechposition, insbesondere nach einer erfolgten Produktausschüttung, wieder, vorzugsweise vollständig in das distale Ende der Injektionsvorrichtung, d.h., in eine Endposition der Injektionsnadel einziehbar. In der Endposition aber auch in der Ausgangsposition besteht der Vorteil, dass man sich durch die Nadel nicht unbeabsichtigt stechen kann.

Ferner umfasst die Vorrichtung einen Schaltnocken, der axial fest zu der Injektionsnadel angeordnet ist und in der Einstechposition der Nadel in ein Sperrfenster eingreift. Der Eingriff dient insbesondere dazu, um eine Rückzugsbewegung der Injektionsnadel in das Innere der Injektionsvorrichtung zu verhindern. Dies ist insbesondere gewünscht, wenn eine Produktausschüttung noch nicht vollständig erfolgt ist. Bevorzugt ist der Schaltnocken mittelbar mit der Injektionsnadel verbunden. Z.B. kann der Schaltnocken an einer Halterung für das Produktbehältnis, was bevorzugt ist, oder an dem Produktbehältnis gebildet sein. Insbesondere kann der Schaltnocken an einem Teil der Vortriebsstruktur, die z.B. wenigstens das Produktbehältnis und die Halterung für das Produktbehältnis umfasst, gebildet sein. Bevorzugt ist der Schaltnocken federnd an einem Teil der Vortriebsstruktur gebildet. Beispielsweise ist der Schaltnocken federnd über einen elastischen Arm mit dem Teil der Vortriebsstruktur insbesondere einteilig verbunden. Dies soll jedoch andere federnde Anordnungen nicht ausschließen.

Das Sperrfenster befindet sich vorzugsweise radial nach außen vom Schaltnocken. Insbesondere kann der Schaltnocken mit einer radial nach außen gerichtete Bewegung in das Sperrfenster eingreifen. Bevorzugt wird das Sperrfenster mit zwei relativ zueinander bewegbaren Teilen distal und proximal begrenzt. Insbesondere begrenzen das Gehäuse oder ein gehäusefestes Element das Sperrfenster distal und die Betätigungshülse das Sperrfenster proximal.

Die in Längsrichtung der Injektionsvorrichtung weisende Breite des Sperrfensters ist veränderbar, so dass je nach Breite des Sperrfensters der Schaltnocken in den Eingriff mit dem Sperrfenster bewegbar ist oder aus dem Eingriff mit dem Sperrfenster bewegbar ist. Mit anderen Worten ist die Breite des Sperrfensters dynamisch. Somit kann das Sperrfenster beispielsweise komplett verschwinden, wenn dessen distale und proximale Begrenzungen aneinander stoßen.

Erfindungsgemäß ist der Schaltnocken mittels der Betätigungshülse aus dem Eingriff mit dem Sperrfenster bewegbar. Hierdurch wird die Injektionsnadel für die Rückzugbewegung aus der Einstechposition in die Endposition freigegeben. Da der Verwender der Vorrichtung über den Anpressdruck der Injektionsvorrichtung an die Injektionsstelle willensabhängig die Position der Betätigungshülse steuern kann, kann er damit den Zeitpunkt, zu dem die Injektionsnadel in das Gehäuse eingezogen wird, selbst bestimmen. Hierzu braucht der Verwender der Vorrichtung lediglich den Anpressdruck der Injektionsvorrichtung auf die Injektionsstelle zu verringern. Insbesondere wird hierdurch die Betätigungshülse in distale Richtung bewegt.

Der Schaltnocken wird aus dem Eingriff mit dem Sperrfenster mit der Bewegung der Betätigungshülse in distale Richtung bewegt. Durch diese Bewegung wird die in Längsrichtung der Injektionsvorrichtung weisende Breite des Sperrfensters so verringert, insbesondere beseitigt, dass der Schaltnocken aus dem Eingriff mit dem Sperrfenster bewegt wird.

Der Vorteil der willentlichen Steuerung des Zeitpunkt des Nadelrückzugs besteht darin, dass der Verwender der Vorrichtung selbst bestimmen kann, insbesondere anhand von Erfahrungswerten oder in Abhängigkeit der Viskosität der Flüssigkeit, wie lange die Nadel nach der Produktausschüttung zur vollständigen Produktausschüttung in dem Gewebe verbleiben soll. Ein Faustwert, der jedoch nur beispielhaft genannt wird, ist eine Zeitdauer von 5 Sekunden. Durch diese Wartezeit kann sichergestellt werden, dass sich das flüssige Produkt im Gewebe besser verteilt und das Produkt aufgrund der Trägheit der Produktausschüttung vollständig ausgeschüttet wurde. Dies bietet zum einen wirtschaftlichen Vorteile und zum anderen Vorteile bei der Bestimmung der tatsächlich verabreichten Produktdosis.

Vorzugsweise sollten, damit der Nadelrückzug erfolgen kann, wenigstens zwei Voraussetzungen erfüllt sein, nämlich dass die Produktausschüttung beendet ist, und die Betätigungshülse in distale Richtung bewegt worden ist.

Vorzugsweise weist der Schaltnocken eine in proximale Richtung weisende Anschlagfläche auf, die mit der Betätigungshülse so zusammenwirken kann, dass der Schaltnocken von der Betätigungshülse bei einer Bewegung der Betätigungshülse in distale Richtung mitgenommen werden kann, wenn sich der Schaltnocken in dem Sperrfenster befindet. Bevorzugt ist daher auch, dass eine Bewegung des Schaltnockens in proximale Richtung gesperrt ist, wenn die Anschlagfläche des Schaltnockens mit der proximalen Begrenzung des Sperrfensters in einem Anschlag ist. Dies verhindert, dass die Nadel und insbesondere auch die gesamte Vortriebsstruktur in proximale Richtung verschoben wird.

Insbesondere kann der Schaltnocken in distaler Richtung so geformt sein, dass er von dem Gehäuse oder dem gehäusefesten Element aus dem Eingriff mit dem Sperrfenster bewegbar ist oder/und dass er von der Betätigungshülse aus dem Eingriff mit einer von der Betätigungshülse gebildeten Aussparung bewegbar ist. Beispielsweise kann eine solche Ausformung eine in distale Richtung weisende schräge Fläche sein, die entsprechend dem Prinzip einer schiefen Ebene den Schaltnocken aus dem Sperrfenster bewegt. Bevorzugt ist die Bewegung mit dem der Schaltnocken aus dem Eingriff mit dem Sperrfenster bewegt wird, eine radiale, insbesondere eine radial nach innen gerichtete Bewegung des Schaltnockens.

Insbesondere in der Ausgangsposition der Vorrichtung kann der Schaltnocken sich in einem Eingriff mit einer von der Betätigungshülse gebildeten Aussparung befinden. Diese Aussparung ist vorzugsweise proximal des Sperrfensters angeordnet. Die Aussparung wird sowohl distal als auch proximal von der Betätigungshülse begrenzt. Die Aussparung kann ein Durchbruch oder eine Tasche sein. Bevorzugt kann der Schaltnocken aus dem Eingriff mit der Aussparung mit einer Bewegung des Schaltnockens in distale Richtung gebracht werden, wie es beispielsweise bei der Bewegung der Nadel aus der Ausgangsposition in die Einstechposition der Fall ist.

Insbesondere wird bevorzugt, dass sich der Schaltnocken bei einer in das distale Ende der Injektionsvorrichtung zurückgezogenen Position, insbesondere der Ausgangsposition und/oder der Endposition der Injektionsnadel, in einem Eingriff mit der proximal des Sperrfensters gebildeten Aussparung befindet.

Vorzugsweise weist das Gehäuse oder ein gehäusefestes Element eine radial nach innen weisende Abragung auf, die in einen von der Wandung der Betätigungshülse gebildeten Durchbruch so weit eingreift, dass die radial nach innen weisende Innenfläche der Abragung in etwa bündig mit der radial nach innen weisenden Innenfläche der Betätigungshülse ist. Insbesondere bildet diese Abragung die distale Begrenzung für das Sperrfenster. Aufgrund der Bündigkeit der Innenflächen kann der Sperrnocken problemlos von der Innenfläche der Abragung auf die Innenfläche der Betätigungshülse gleiten, wie das z.B. der Fall ist, wenn die Nadel aus der Einstechposition in die Endposition bewegt wird. Dabei ist das Sperrfenster geschlossen, d. h., dass dessen proximale Begrenzung an die distale Begrenzung anschlägt, so dass die Fensterbreite null ist.

Besonders bevorzugt ist der Schaltnocken mit einer Rückzugsfeder gekoppelt, mit dem der Schaltnocken und die damit axial fest verbundene Injektionsnadel sowie insbesondere die gesamte Vortriebsstruktur, in proximale Richtung bewegbar sind. Insbesondere ist der Schaltnocken mittelbar mit der Rückzugsfeder gekoppelt, wie z.B. über die Halterung für das Produktbehältnis und eine Funktionshülse, an der sich die Rückzugsfeder, insbesondere mit ihrem proximalen Ende abstützt. Insbesondere können die Halterung und die Funktionshülse axial fest miteinander gekoppelt sein, so dass sie sich wie ein einziges Teil verhalten können.

Vorzugsweise ist die Rückzugsfeder eine wendelförmige Druckfeder, die mit einer Bewegung der Funktionshülse in distale Richtung vorspannbar und mit einer Bewegung der Funktionshülse in proximale Richtung entspannbar ist. Besonders bevorzugt ist die Rückzugsfeder von einer Vortriebsfeder vorspannbar. Die Vortriebsfeder kann zum Vortrieb der Nadel aus der Ausgangsposition in die Einstechposition und vorzugsweise auch zur Produktausschüttung dienen. In bevorzugten Ausführungsformen ist daher eine einzige Vortriebsfeder ausreichend.

In bevorzugten Ausführungen kann die Injektionsvorrichtung ein Sperrelement umfassen, welches mit dem Schaltnocken über die Vortriebsstruktur axial fest verbunden ist. Insbesondere ist das Sperrelement über die Funktionshülse und die Halterung für das Produktbehältnis mit dem Schaltnocken verbunden. Hierdurch weisen das Sperrelement und der Schaltnocken zueinander einen fest definierten axialen Abstand auf, der während der gesamten Schaltvorgänge in der Injektionsvorrichtung nicht verändert wird. Das Sperrelement kann in der Einstechposition der Injektionsnadel in einen Eingriff mit der proximal des Sperrfensters von der Betätigungshülse gebildeten Aussparung bewegbar sein. Insbesondere kann das Sperrelement bei Beendigung der Einstechbewegung in den Eingriff mit der Aussparung bewegt werden. Hierdurch wird die Injektionsnadel für einen Rückzug aus der Einstechposition in proximale Richtung blockiert. Insbesondere kann das Sperrelement bei der Bewegung in den Eingriff einen Sperreingriff mit einer auf den Kolben wirkenden Kolbenstange lösen, so dass hierdurch gleichzeitig die Kolbenstange für eine Ausschüttbewegung von der Vortriebsfeder angetrieben wird.

Des Weiteren kann durch die Bewegung des Sperrelements in den Eingriff mit der Aussparung die Rückzugsfeder von der Vortriebsfeder entkoppelt werden, nachdem die Vortriebsfeder bei der Einstechbewegung die Rückzugsfeder vorgespannt hat.

Besonders bevorzugt befinden in der Einstechposition der Nadel und insbesondere vor einer vollständig erfolgten Produktausschüttung, d.h., während der Ausschüttbewegung, der Schaltnocken sich in einem Eingriff mit dem Sperrfenster und das Sperrelement sich in einem Eingriff mit der von der Betätigungshülse gebildeten Aussparung. Während diesem Zustand der Injektionsvorrichtung befindet sich zwischen der proximalen Begrenzung des Sperrfensters und der in proximale Richtung weisenden Anschlagfläche des Schaltnockens ein kleiner Abstand, insbesondere von 0,3 bis 3 mm. Das Sperrelement befindet sich hierbei mit seiner in proximale Richtung weisenden Anschlagfläche in Berührung mit der proximalen Begrenzung der Aussparung der Betätigungshülse. Besonders bevorzugt ist der axiale Abstand, der zwischen der in proximalen Richtung weisenden Anschlagfläche des Schaltnockens und der in proximale Richtung weisenden Anschlagfläche des Sperrelements herrscht, größer, insbesondere um den kleinen Abstand größer als der axiale Abstand zwischen der proximalen Begrenzung der Aussparung der Betätigungshülse und der proximalen Begrenzung des Sperrfensters. Durch den genannten kleinen Abstand kann ein haptisches, insbesondere taktiles, oder akustisches Signal erzeugt werden, das die Vollendung der Produktausschüttung anzeigt. Bevorzugt ist, dass die Vortriebsstruktur, und damit das Sperrelement und der Schaltnocken von der Rückzugsfeder um den Betrag des kleinen Abstands in proximale Richtung bewegbar ist. Hierdurch schlägt die in proximale Richtung weisende Anschlagfläche des Schaltnockens schlagartig an die proximale Begrenzung des Sperrfensters an, wodurch das Signal erzeugt wird.

Bevorzugt kann das Sperrelement nach Beendigung der Produktausschüttung aus dem Eingriff mit der Aussparung ausrasten, wodurch die Rückzugsfeder sich um den kleinen Abstand zur Erzeugung des Signals entspannen kann. Besonders bevorzugt wird, dass ein Ausrasten des Sperrelements aus dem Eingriff mit der Aussparung blockiert ist, insbesondere von der äußeren Umfangsfläche der sich am Sperrelement vorbeibewegenden Kolbenstange.

Offenbart wird auch ein Verfahren zum Einziehen einer aus dem distalen Ende der Injektionsvorrichtung ausgefahrenen Injektionsnadel in das distale Ende, wobei die Betätigungshülse aus dem distalen Ende der Injektionsvorrichtung bewegt und dabei ein in ein Sperrfenster eingreifender Schaltnocken von der Betätigungshülse mitgenommen wird. Bei der Mitnahmebewegung wird der Schaltnocken aus dem Sperrfenster bewegt. Der mit der Injektionsnadel fest verbundene Schaltnocken wird in proximale Richtung bewegt, so dass die ausgefahrene Injektionsnadel in das distale Ende der Injektionsvorrichtung eingezogen wird. Das Verfahren ist vollständig, d. h. mit all seinen Verfahrensschritten, außerhalb des menschlichen oder tierischen Körpers durchführbar.

Vorteilhafte Verfahrensschritte ergeben sich aus der Arbeitsweise der Vorrichtung.

Die Erfindung wird nun anhand von Figuren beschrieben.

Es zeigen:
- Figuren 1a und 1b: Schnittdarstellung einer erfindungsgemäßen Injektionsvorrichtung mit einer aufgesetzten Kappe, wobei Figur 1b eine gegenüber Figur 1a um 90° um die Längsachse gedrehte Ansicht ist,
- Figuren 2a und 2b: Schnittdarstellungen der Injektionsvorrichtung mit einer abgenommenen Kappe, wobei Figur 2b eine gegenüber Figur 2a um 90° um die Längsachse gedrehte Ansicht ist,
- Figuren 3a und 3b: Schnittdarstellungen der erfindungsgemäßen Injektionsvorrichtung in einem aktivierten Zustand, wobei Figur 3b eine gegenüber Figur 3a um 90° um die Längsachse gedrehte Ansicht ist,
- Figuren 4a und 4b: Schnittdarstellungen der in erfindungsgemäßen Injektionsvorrichtung in einem ausgelösten Zustand, wobei Figur 4b eine gegenüber Figur 4a um 90° um die Längsachse gedrehte Ansicht ist,
- Figuren 5a und 5b: Schnittdarstellungen der erfindungsgemäßen Injektionsvorrichtung in einem eingestochenen Zustand, wobei Figur 5b eine gegenüber Figur 5a um 90° um die Längsachse gedrehte Ansicht ist,
- Figuren 6a und 6b: Schnittdarstellungen der erfindungsgemäßen Injektionsvorrichtung in einem ausgeschütteten Zustand, wobei Figur 6b eine gegenüber Figur 6a um 90° um die Längsachse gedrehte Ansicht ist,
- Figuren 7a und 7b: Schnittdarstellungen der erfindungsgemäßen Injektionsvorrichtung in einem Zustand, in dem die Injektionsvorrichtung ein das Ende der Ausschüttung signalisierendes Klickgeräusch abgegeben hat, wobei Figur 7b eine gegenüber Figur 7a um 90° um die Längsachse gedrehte Ansicht ist,
- Figuren 8a und 8b: Schnittdarstellungen der erfindungsgemäßen Injektionsvorrichtung bei der ein Rückzug der Injektionsnadel aktiviert ist, wobei Figur 8b eine gegenüber Figur 8a um 90° um die Längsachse gedrehte Ansicht ist,
- Figuren 9a und 9b: Schnittdarstellungen der erfindungsgemäßen Injektionsvorrichtung in einem Endzustand, wobei Figur 9b eine gegenüber Figur 9a um 90° um die Längsachse gedrehte Ansicht ist,
- Figur 10: eine Schnittdarstellung der Signalisierungseinheit aus den Figuren 1 bis 9,
- Figur 11: eine perspektivische Ansicht der Signalisierungseinheit aus Figur 10,
- Figur 12: eine erfindungsgemäße Injektionsvorrichtung mit einer anderen Ausführungsform einer Signalisierungseinheit,
- Figur 13: eine Schnittdarstellung der Signalisierungseinheit aus Figur 12,
- Figur 14: eine weitere Schnittdarstellung der Signalisierungseinheit aus Figur 12, und
- Figur 15 und 16: eine weitere Ausführungsform einer erfindungsgemäßen Signalisierungseinheit.

Sofern nichts anderes angegeben wird, bezeichnen gleiche Bezugszeichen die gleichen Teile.

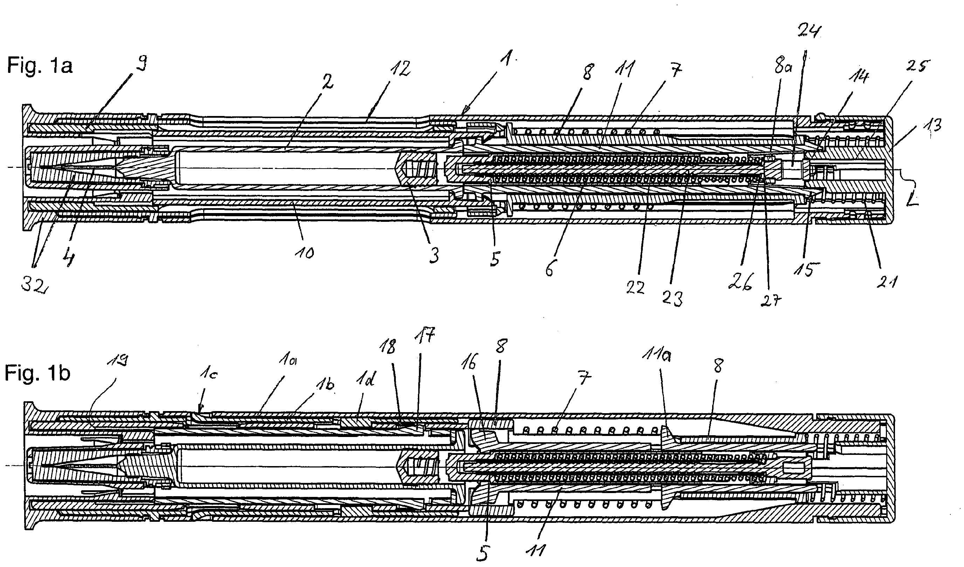

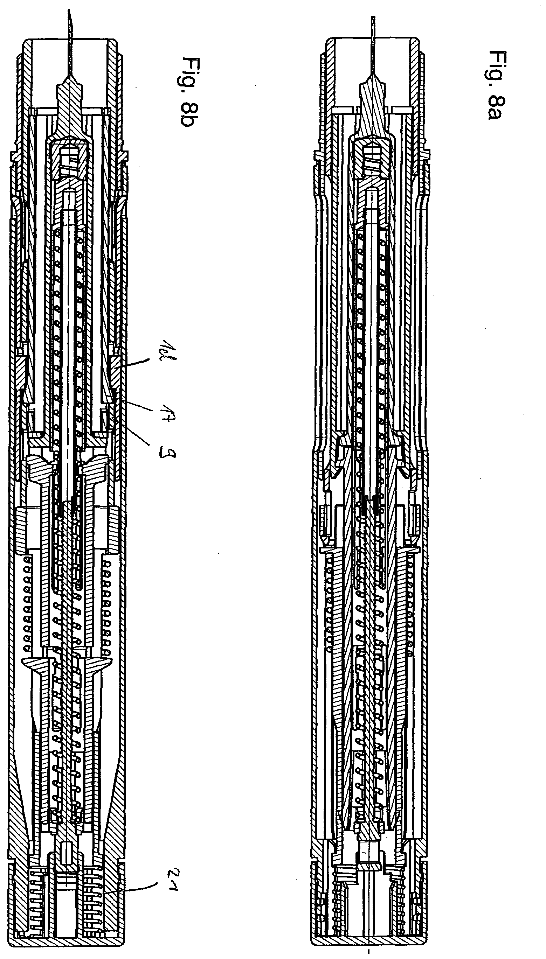

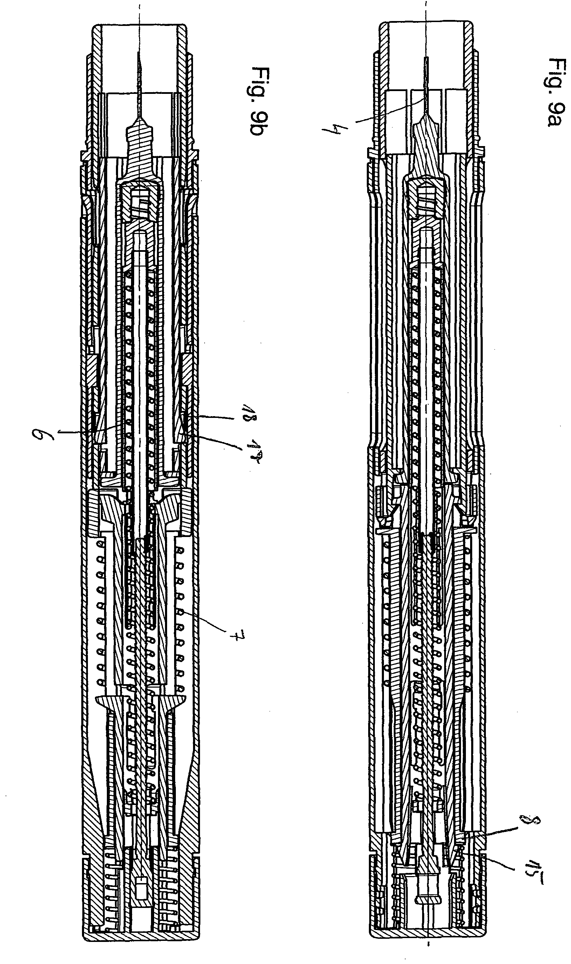

Figuren 1a, 1b bis 9a, b zeigen eine Injektionsvorrichtung einer bevorzugten Ausführungsform. Insbesondere bezugnehmend auf die Figuren 1a und 1b weist die Injektionsvorrichtung ein Gehäuse 1 auf, das aus einem proximalen Gehäuseteil la und einem mit dem proximalen Gehäuseteil mit einer Rastverbindung 1c axial fest verbundenes, distales Gehäuseteil 1b. Die Rastverbindung 1c wird gebildet aus einem im proximalen Gehäuseteil enthaltenen Fenster, in welches eine vom distalen Gehäuseteil 1b gebildete elastische Zunge eingeschnappt ist.

Im Gehäuse 1 ist ein Produktbehältnis 2 aufgenommen, an dessen distalem Ende sich eine Injektionsnadel 4 zur Ausschüttung eines in dem Produktbehältnis 2 enthaltenen flüssigen Produkts befindet. Am proximalen Ende weist das Produktbehältnis 2 einen verschiebbaren Kolben 3 auf, dessen Bewegung relativ zum Produktbehältnis 2 und in Richtung Injektionsnadel 4 eine Produktausschüttung bewirkt, weshalb auch von einer Ausschüttbewegung gesprochen werden kann. Das Produktbehältnis 2 ist so in der Vorrichtung aufgenommen, dass es in distale Richtung verschiebbar ist, so dass die Injektionsnadel 4 aus dem distalen Ende der Injektionsvorrichtung hervortritt. Deshalb kann hier von einer Einstechbewegung die Rede sein. Das Produktbehältnis 2 ist axial fest mit einer Halterung 10 für das Produktbehältnis 2 verbunden. Das Gehäuse 1, insbesondere dessen distale und proximale Gehäuseteile 1a, 1b weisen ein Sichtfenster 12 auf, durch das der Verwender der Injektionsvorrichtung einen Blick auf das Produktbehältnis 2 werfen kann. Die Halterung 10 umgibt das Produktbehältnis 2 hülsenförmig, so dass es, um den Blick auf das Behältnis 2 freizugeben, entweder selbst ein Sichtfenster oder wie in diesem Beispiel ein transparentes Material aufweist. Das Produktbehältnis 2 ist mit einer proximal von ihr angeordneten Funktionshülse 11 am proximalen Ende der Halterung 10 gebildeten Klammer axial fest verbunden. Das Produktbehältnis 2 weist an seinem proximalen Ende einen radial abstehenden Kragen auf, der von der Klammer gefasst wird. Die Funktionshülse 11 weist an ihrem distalen Ende auch einen radial abstehenden Kragen auf, der ebenfalls von der Klammer umgriffen wird. Somit sind Produktbehältnis 10, Funktionshülse 11 und Halterung 10 axial fest miteinander verbunden, so dass sie sich wie ein einziges Teil bewegen lassen. Im Folgenden wird diese Kombination als Vortriebsstruktur 2, 10, 11 bezeichnet.

Die Funktionshülse 11 umgibt eine Kolbenstange 5, die für eine Produktausschüttung auf den Kolben 3 wirken kann. Die Kolbenstange 5 weist einen hülsenförmigen Teil auf, der eine Vortriebsfeder 6 umgibt, wobei sich die Vortriebsfeder 6 distal an der Kolbenstange 5 und proximal an einer Schalthülse 8, insbesondere an einem daran gebildeten Sockel 8a abstützt.

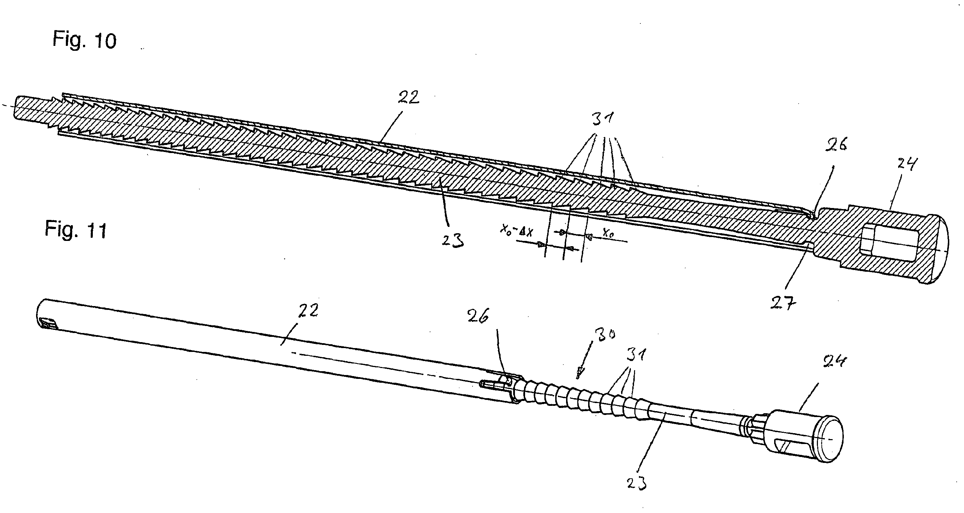

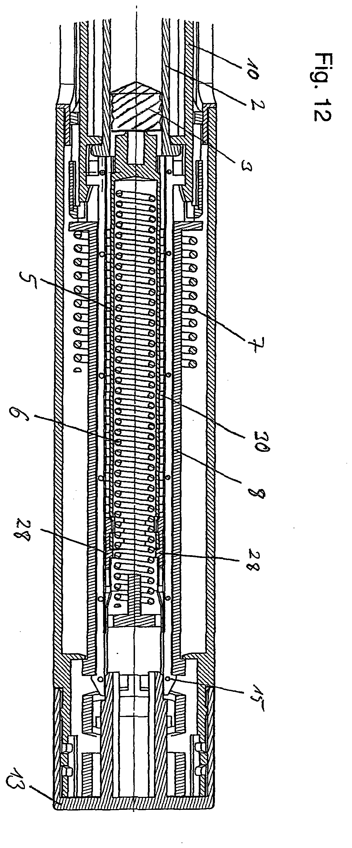

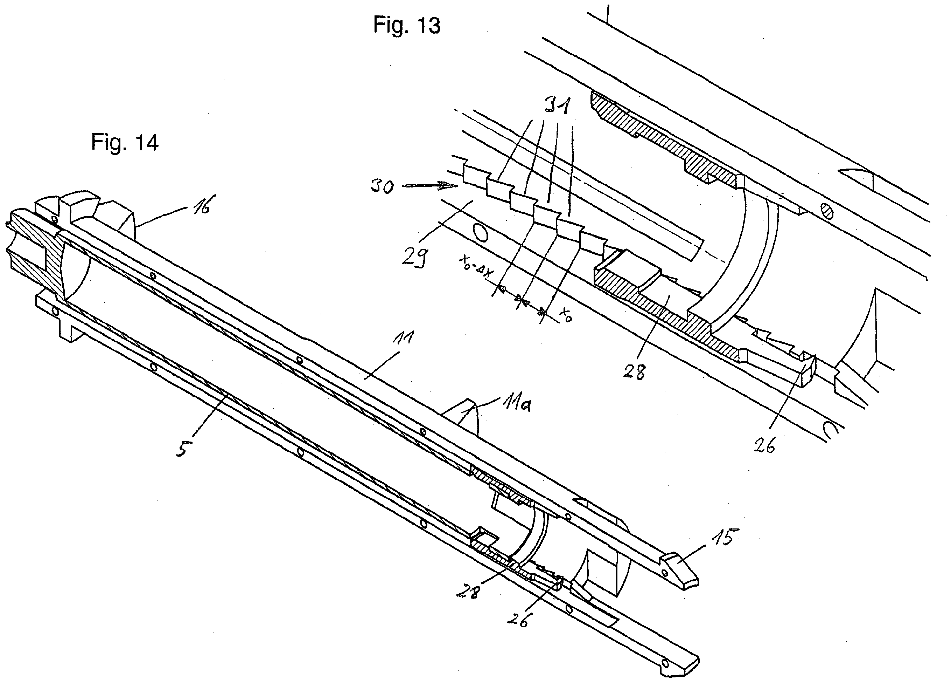

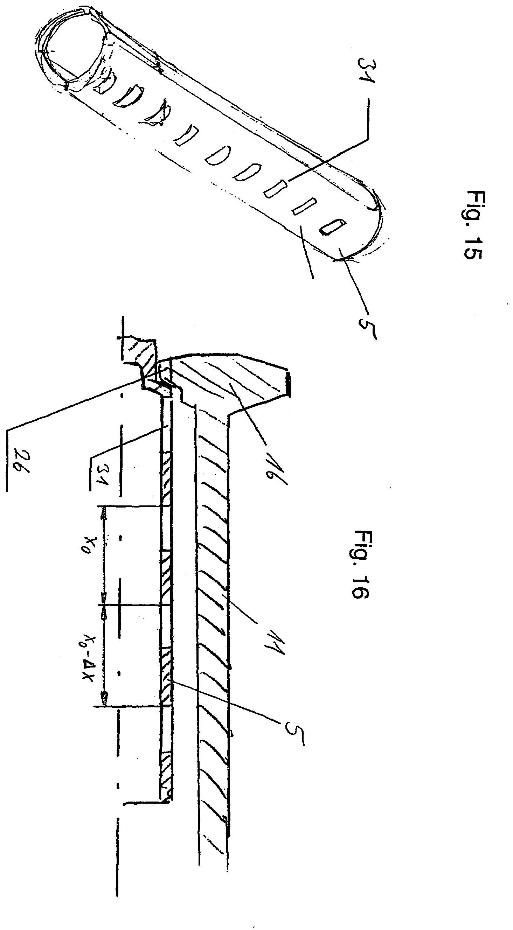

An der Kolbenstange 5 ist eine Signalisierungseinheit angeordnet, mit der für den Einstechvorgang und/oder den Ausschüttvorgang ein, vorzugsweise mindestens drei oder mehrere haptische und/oder akustische Signale erzeugbar sind. Die Signalisierungseinheit umfasst eine mit der Schalthülse 8 verbundene Rasterstange 23 und eine die Rasterstange 23 umgebende Eingriffshülse 22, welche mit der Kolbenstange 5 axial fest verbunden, insbesondere verrastet ist. Die Eingriffshülse 22 weist ein Eingriffselement 26 auf, das in eine von der Rasterstange 23 gebildete Nut 27 eingreift. Die Rasterstange 23 weist an ihrem proximalen Ende einen Kopf 24 auf, der in einer von dem Aktivierungselement 13 gebildeten Gleitführung 25 in proximale Richtung bewegbar ist. Der Kopf ist mit seinem distalen Ende in einem Eingriff mit einem von der Schalthülse 8 gebildeten Sockel 8a, wobei der Eingriff verhindert, dass der Kopf 24 und damit die Rasterstange 23 in distale Richtung relativ zur Schalthülse 8 bewegbar ist. Die genaue Funktionsweise dieser Anordnung wird später unter Bezugnahme auf die Figuren 10 und 11 erklärt, in denen die in den Figuren 1 bis 9 gezeigte Signalisierungseinheit im Detail dargestellt ist. Alternativ kann die Signalisierungseinheit aus den Figuren 10 und 11 durch eine andere Signalisierungseinheit gemäß Figuren 12 bis 14 und noch einer anderen Signalisierungseinheit gemäß Figuren 15 und 16 ersetzt werden. Die in den Figuren 1 bis 9 gezeigt Injektionsvorrichtung muss hierzu nicht wesentlich geändert werden.

In dem in den Figuren 1a und 1b gezeigten Ausgangszustand der Injektionsvorrichtung ist die Vortriebsfeder 6 vorgespannt, so dass sie die Nadel 4 und insbesondere die Vortriebsstruktur 2, 10, 11 für eine Einstechbewegung vorantreiben und den Kolben 3 für eine Ausschüttbewegung verschieben kann. Die Funktionshülse 11 weist ein Sperrelement 16 auf, an dem eine radial nach innen gerichtete Schulter ausgebildet ist, die im Ausgangszustand mit einer am distalen Ende der Kolbenstange 5 gebildeten radial nach außen stehenden Schulter zusammenwirkt, so dass die Kolbenstange 5 für eine Bewegung relativ zur Funktionshülse 11 gesperrt ist. Das Sperrelement 16 wird von einer radial nach innen weisenden Fläche der Schalthülse 8 in dem Eingriff mit der Kolbenstange 5 gehalten. Bevorzugt ist das Sperrelement 16 über einen Federarm elastisch mit der Funktionshülse 11 verbunden, insbesondere einteilig. Die federnde Anordnung kann so gestaltet sein, dass das Sperrelement 16 dazu tendiert, sich radial nach außen zu bewegen, wobei dies durch die radial nach innen weisende Fläche der Schalthülse 8 verhindert wird.

Die Funktionshülse 11 weist an ihrem proximalen Ende mindestens ein Schnappelement 15 auf, welches im Ausgangszustand zur Verhinderung einer Bewegung der Funktionshülse 11 und damit der Vortriebsstruktur 2, 10, 11 in die Schalthülse 8 einschnappt. Hierdurch kann die vorgespannte Feder 6 sich noch nicht entspannen und die Vortriebsstruktur 2, 10, 11 noch nicht in distale Richtung bewegen.

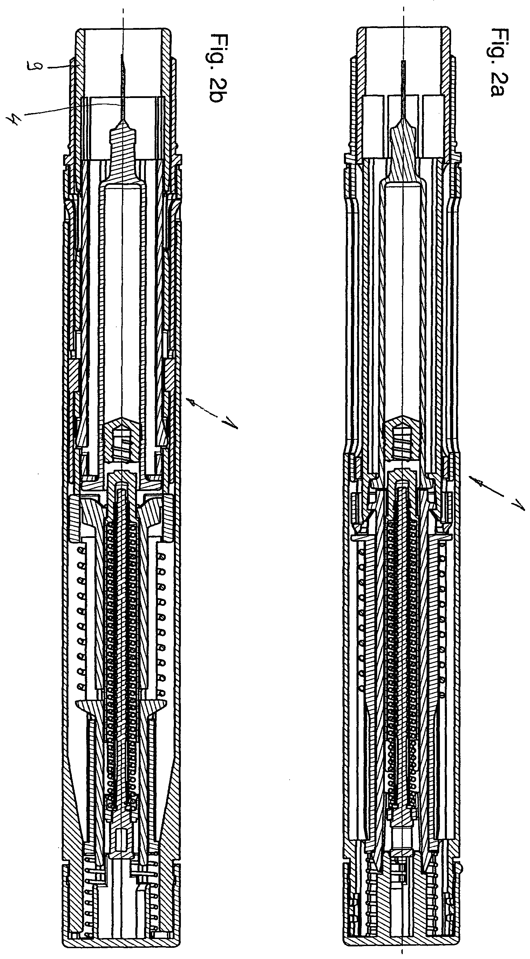

Die Injektionsvorrichtung weist am proximalen Ende ihres Gehäuses 1 ein Aktivierungselement 13 auf, welches zum Gehäuse 1 axial fest und drehbar angeordnet ist. Das Aktivierungselement 13 nimmt eine Rückstellfeder 21 auf, die sich distal am proximalen Ende der Schalthülse 8 und proximal an dem Aktivierungselement 13 abstützt. Die Rückstellfeder 21 hat die Aufgabe, die Schalthülse 8 und eine axial auf die Schalthülse 8 wirkende Betätigungshülse 9 mit einer in distale Richtung wirkenden Kraft zu beaufschlagen, so dass Schalthülse 8 und Betätigungshülse 9 in distale Richtung gedrückt werden. Das Aktivierungselement 13 weist eine Aktivierungssicherung 14 auf, die in den in den Figuren 1a, 1b, 2a und 2b gezeigten Schaltzuständen der Injektionsvorrichtung das Schnappelement 15 so hintergreift, dass das Schnappelement 15 für eine Bewegung aus dem Eingriff mit der Schalthülse 8 blockiert bzw. gesichert ist. Somit kann vorteilhaft ein versehentliches Auslösen der Injektionsvorrichtung verhindert werden. Durch Drehung des Aktivierungselements 13 z. B. um 90° relativ zum Gehäuse 1 kann die Aktivierungssicherung aus dem Eingriff mit dem Schnappelement 15 bewegt werden.

Eine in Längsrichtung der Vorrichtung wirkende Rückzugsfeder 7 stützt sich distal an der Schalthülse 8 und proximal an der Funktionshülse 11 ab. Wie in diesem Beispiel gezeigt wird, umgibt die Rückzugsfeder 7 die Schalthülse 8 und die Funktionshülse 11. Die Rückzugsfeder 7 stützt sich proximal insbesondere an einem von der Funktionshülse 11 gebildeten Bund 11a ab, der radial nach außen durch einen in der Schalthülse 8 gebildeten Durchbruch greift. In bestimmten Schaltpositionen kann daher die Rückzugsfeder 7 eine Relativbewegung zwischen der Schalthülse 8 und der Funktionshülse 11 bewirken. Die Rückzugsfeder 7 ist eine Druckfeder, welche die Funktionshülse 11 relativ zur Schalthülse 8 in proximale Richtung bewegen kann. Die Rückzugsfeder 7 ist nicht oder bevorzugt nur mit einer geringen Vorspannkraft vorgespannt. Beispielsweise in dem in den Figuren 1a und 1b gezeigten Zustand der Injektionsvorrichtung ist die Vorspannkraft der Rückzugsfeder 7 geringer als die Vorspannkraft der Vortriebsfeder 6.

Distal der Schalthülse 8 ist die Betätigungshülse 9 relativ zum Gehäuse 1 bewegbar angeordnet. Die Schalthülse 8 und die Betätigungshülse 9 können sich jeweils gegenseitig mit einer Druckkraft beaufschlagen, insbesondere miteinander verrastet sein und sich dadurch verschieben. Damit die Betätigungshülse 9 die Sicht auf das Produktbehältnis 2 nicht blockiert, weist sie im Bereich des Fensters 12 ebenfalls ein Fenster auf. Alternativ kann die Betätigungshülse 9 aus einem transparenten Material gebildet sein. Die Betätigungshülse 9 wird im Ausgangszustand von der Rückstellfeder 21 über die Schalthülse 8 distal über das distale Ende des Gehäuses 1 vorgeschoben. Das distale Ende der Betätigungshülse 9 dient dazu, an eine Injektionsstelle eines Patienten angesetzt zu werden.

Die Halterung 10 weist einen Schaltnocken 17 auf, der in eine Aussparung 18 der Betätigungshülse 9 eingreift, die, wie in diesem Beispiel gezeigt wird, ein Durchbruch sein kann. Der Schaltnocken 17 ist elastisch mit der Halterung 10, wie z. B. über einen federnden Arm, insbesondere einteilig verbunden. Der Schaltnocken 17 ist vorzugsweise so vorgespannt, dass er dazu neigt, in die Aussparung 18 einzugreifen bzw. sich radial nach außen zu bewegen. Der radial von der Halterung 10 nach außen abragende Schaltnocken 17 weist distal eine schräge Fläche auf, die dabei mitwirken kann, den Schaltnocken 17 aus dem Eingriff mit der Aussparung 18 zu drücken. Ferner weist der Schaltnocken 17 proximal eine quer, insbesondere senkrecht zur Längsrichtung stehende Anschlagfläche auf, die mit der proximalen Begrenzung der Aussparung 18 in einen Axialanschlag geraten kann, durch den der Schaltnocken 17 nicht aus der Aussparung 18 bewegt werden kann.

Die Betätigungshülse 9 weist einen Axialanschlag 19 auf, an dem das distale Ende der Halterung 10 am Ende einer Einstechbewegung anschlagen kann.

Am distalen Ende der Injektionsvorrichtung ist, wie in den Figuren 1a und 1b gezeigt wird, eine Kappe 32 angeordnet, welche das innere der Injektionsvorrichtung vor Verschmutzung schützt, insbesondere die Nadel 4 steril hält. Vor Verwendung der Injektionsvorrichtung wird die Kappe 32 abgezogen, so dass insbesondere die Nadel 4 und die Betätigungshülse 9 freiliegen, wie in den Figuren 2a und 2b gezeigt wird. Der in den Figuren 2a und 2b gezeigte Zustand der Injektionsvorrichtung unterscheidet sich von dem in den Figuren 1a und 1b gezeigten Zustand lediglich dadurch, dass die Kappe 32 abgenommen ist.

Die Kraft, die bei beim Abzug der Nadelkappe 32 auf die Injektionsvorrichtung ausgeübt wird, wird über die Halterung 10 auf die Funktionshülse 11 geleitet und dort über die Schnapper 15 auf die Schalthülse 8 übergeben, welche sich an der Betätigungshülse 9 abstützt. Die Betätigungshülse 9 ist ihrerseits mit dem Gehäuse 1 über eine vom distalen Gehäuseteil 1 gebildete Abragung 1d verrastet, so dass das Abziehen der Kappe 32 von der Injektionsvorrichtung keine unerwünschte Auswirkung auf die Mechanik ausübt.

In dem in den Figuren 2a und 2b gezeigten Schaltzustand kann die Betätigungshülse 9 nicht oder nur sehr geringfügig in das distale Ende der Injektionsvorrichtung hineingeschoben werden, da diese Verschiebebewegung über die Schalthülse 8 an den Schnapper 15 weitergeleitet wird, wobei der Schnapper 15 an einer Bewegung in proximale Richtung durch das Aktivierungselement 13 gehindert wird.

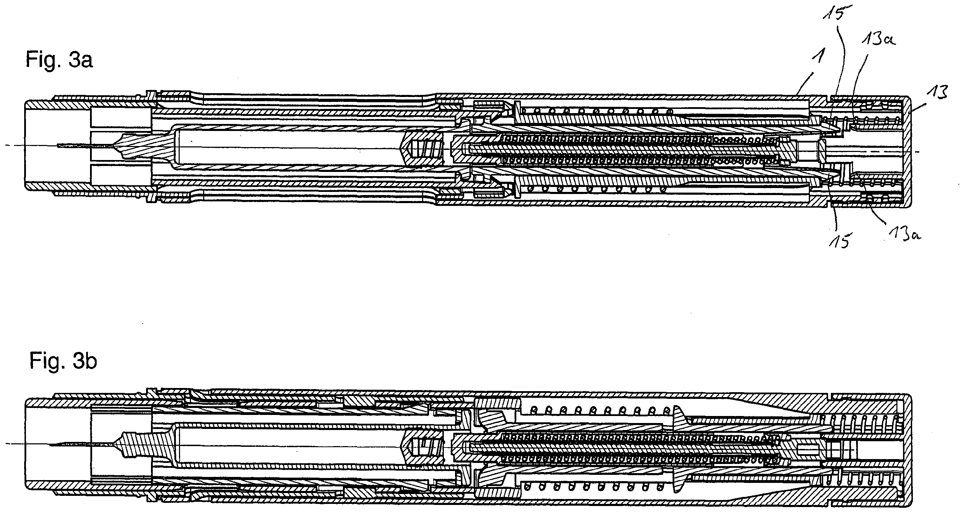

In den Figuren 3a und 3b wird die Injektionsvorrichtung in einem aktivierten Zustand gezeigt, d.h., dass die Injektionsvorrichtung auslösbar ist. Aktiviert bzw. entriegelt wird die Injektionsvorrichtung mit einer Drehbewegung des Aktivierungselements 13 z. B. um 90°. Hierbei werden die Schnappelemente 15 für eine radial nach innen gerichtete Bewegung dadurch freigegeben, dass die Aktivierungssicherung 14 aus dem Eingriff mit den Schnappelementen 15 bewegt, insbesondere gedreht wird. Somit besteht Raum für die Schnappelemente 15 nach innen hin ausgelenkt zu werden. Ferner weist das Aktivierungselement 13 wie das Schnappelement 15 einen Aktivierungsnocken 13a auf, der durch die Drehbewegung des Aktivierungselements 13 in eine axiale Flucht mit dem Schnappelement 15 gebracht wird. Das Schnappelement 15 weist proximal und der proximal davon angeordnete Aktivierungsnocken 13a weist distal eine Kontur auf, welche bei der Bewegung des Schnappelements 15 in einem Eingriff mit dem Aktivierungsnocken 13 das Schnappelement 15 radial nach innen auslenken kann. In diesem Beispiel sind die Konturen zwei aufeinander abgleitende schiefe Ebenen.

Zur Auslösung der Injektionsvorrichtung setzt der Verwender der Vorrichtung diese mit dem distalen Ende auf die vorzugsweise vorher desinfizierte Injektionsstelle. Hierdurch wird die Betätigungshülse 9 relativ zum Gehäuse 1 in proximale Richtung verschoben, vorzugsweise soweit, bis das distale Ende der Betätigungshülse 9 in etwa bündig mit dem distalen Ende des distalen Gehäuseteils 1b ist. Durch die Bewegung der Betätigungshülse 9 wird die Schalthülse 8 in proximale Richtung mitgenommen, wobei die Schnappelemente 15 mittels der Aktivierungsnocken 13a aus dem Eingriff mit der Schalthülse 8 insbesondere radial nach innen gedrückt werden. Mit der Bewegung der Betätigungshülse 9 in distale Richtung werden, solange die Schnappelemente 15 in die Schalthülse 8 eingeschnappt sind, auch die Elemente der Vortriebsstruktur 2, 10, 11 in proximale Richtung mitgenommen. Da die Kolbenstange 5 mit der Funktionshülse 11 sich in einem Sperreingriff befindet, wird auch die Kolbenstange 5 in proximale Richtung mitgenommen. Ebenso wird die in der Kolbenstange 5 aufgenommene Signalisierungseinheit in proximale Richtung mitgenommen. Der proximal an der Rasterstange 23 gebildete Kopf 24 kann in der vom Aktivierungselement 13 gebildeten Führung 25 entlanggleiten.

Da bei dieser Bewegung noch keine Relativbewegung zwischen der Aktivierungshülse 11 und der Schalthülse 8 stattfinden kann, werden weder die Rückzugsfeder 7 noch die Vortriebsfeder 6 ge- oder entspannt.

Die Kraft, die der Verwender der Vorrichtung auf das Gehäuse 1 ausüben muss, damit die Betätigungshülse 9 in proximale Richtung verschoben wird, bestimmt sich im Wesentlichen über die Kraft der Rückstellfeder 21, gegen die die Schalthülse 8 und die Betätigungshülse 9 bewegt werden. Die Feder 21 ist vorzugsweise eine Druckfeder und aus einem Kunststoffmaterial gebildet. Alternativ sind natürlich auch Federn aus einem Federstahlwerkstoff oder einem anderen Federwerkstoff verwendbar. Die axiale Befestigung des Aktivierungselements 13 an dem Gehäuse 1 besteht in der Gestalt einer Ringschnappverbindung mit dem Gehäuse. Wird die Betätigungshülse 9 nicht ausreichend weit auf die Injektionsstelle gedrückt, so dass die Schnappelemente 15 nicht aus dem Eingriff mit der Schalthülse 8 gelöst sind, erfolgt beim Abnehmen der Injektionsvorrichtung von der Injektionsstelle eine Rückstellung der Auslösemechanik, z.B. der Schalthülse 8 und der Betätigungshülse 9 durch die Rückstellfeder 21.

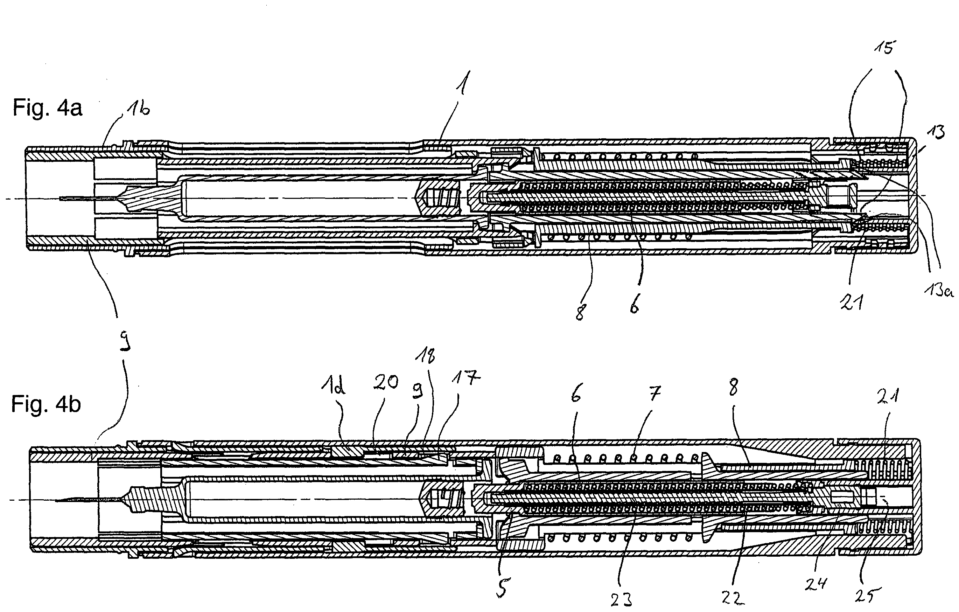

Wie aus Figur 4b erkennbar ist, wird durch die Bewegung der Betätigungshülse 9 in proximale Richtung ein Sperrfenster 20 gebildet, welches distal von dem Gehäuse 1, insbesondere der Abragung 1d und proximal von der Betätigungshülse 9 begrenzt ist. Da bei der Bewegung der Betätigungshülse 9 in proximale Richtung noch keine Relativbewegung zwischen der Vortriebsstruktur 2, 10, 11 und der Betätigungshülse 9 stattfindet, verbleibt der Schaltnocken 17 in der Aussparung 18.

Nachdem die Schnapper 15 aus dem Eingriff mit der Schalthülse 8 ausgerastet sind, kann sich die Vortriebsfeder 6 teilweise entspannen, wodurch die Vortriebsstruktur 2, 10, 11 in distale Richtung verschoben wird. Dabei tritt die Injektionsnadel 4 über das distale Ende der Injektionsvorrichtung hervor. Da bei dieser Einstechbewegung die Funktionshülse 11 relativ zur Schalthülse 8 bewegt wird, wird die Rückzugsfeder 7 komprimiert, d.h. gespannt. Die Federkraft der Vortriebsfeder 6 ist während des gesamten Einstechvorgangs, d.h. auch am Anfang und am Ende des Einstechvorgangs größer als die Federkraft der Rückzugsfeder 7. Dies hat z.B. den Vorteil, dass die Einstechkraft reduziert wird, was zur Schonung der Injektionsvorrichtung beiträgt.

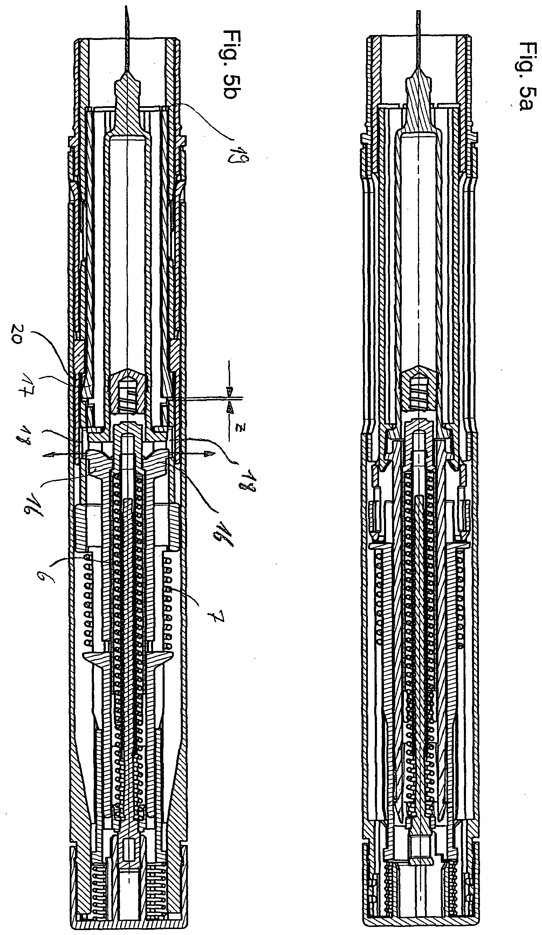

Wie aus den Figuren 5a und 5b, in denen die Situation am Ende der Einstechvorrichtung gezeigt ist, ersichtlich ist, greift das Sperrelement 16 mit einer radial nach außen gerichteten Bewegung, wie mit den Pfeilen in Figur 5b gezeigt werden soll, in die Aussparung 18 ein. Um diesen Eingriff zu verbessern weist das Sperrelement 16 eine radial nach außen gerichtete Abragung auf. Das Sperrelement 16 erfüllt eine Doppelfunktion. Beim Einrasten des Sperrelements 16 in die Aussparung 18 rastet gleichzeitig das Sperrelement 16 mit der nach radial außen gerichteten Bewegung aus der Kolbenstange 5 aus, so dass diese für eine Ausschüttbewegung freigegeben ist. Im Gegenzug wird die Bewegung der Vortriebsstruktur 2, 10, 11 in axiale Richtung, insbesondere in proximale Richtung gesperrt. Durch diesen Vorgang wird die Vorschubfeder 6 von der Rückzugsfeder 7 entkoppelt, d.h., dass die Vorschubfeder 6 in diesem Zustand keinen Einfluss auf die Vorspannung der Rückzugsfeder 7 hat. Es folgt eine Ausschüttbewegung, bei der durch die Signalisierungseinheit ein zeitkonstantes Klickgeräusch abgegeben wird, das vom Verwender der Vorrichtung auch fühlbar ist.

Für den Verwender der Vorrichtung wird durch den Einstechvorgang keine zusätzliche Kraft spürbar. Diese wird durch die Verschnappung zwischen der Betätigungshülse 9 und der Schalthülse 8 gefangen und stützt sich nicht am Gehäuse ab. Die Kraft für den Einstechvorgang wird über die Funktionshülse 11 an den Kragen des Produktbehältnisses 2 geleitet. Somit ist der Einstechvorgang zwangsgesteuert, da die Funktionshülse 11 bis zum Ende der Ausschüttung das Produktbehältnis 2 vorantreibt und die Kolbenstange 5 erst nach dem Eingriff der Sperrelemente 16 in die Aussparungen 18 ausschütten kann. Die Einstechbewegung wird durch den Anschlag 19 an der Betätigungshülse 9 gestoppt.

Bei der Einstechbewegung wird der Schaltnocken 17 aufgrund seiner distalen Gestaltung von der distalen Begrenzung der Aussparung 18 der Betätigungshülse 9 aus dem Eingriff mit der Aussparung 18 gedrückt und in distale Richtung verschoben, so dass er in das Sperrfenster 20 einrastet, wie in den Figuren 5a und 5b gezeigt wird. Das in die Aussparung 18 eingerastete Sperrelement 16 steht mit der proximalen Begrenzung der Aussparung 18 in Kontakt. Da das Sperrelement 16 und der Schaltnocken 17 aufgrund ihrer axial festen Anordnung zueinander in einem definierten Abstand stehen, ist es bevorzugt, dass bei einem im Eingriff mit der Aussparung stehenden Sperrelement 16 zwischen dem proximalen Ende des Schaltnockens und dem distalen Ende des Sperrfensters 20 ein kleiner Abstand besteht, der in diesem Beispiel 0,5 bis 1 mm beträgt. Dieser Abstand wird, wie weiter unten erklärt wird, zur Erzeugung eines haptischen oder akustischen Signals verwendet, welches die vollständige Produktausschüttung signalisieren soll. Der kleine Abstand z entsteht aus der Differenz aus dem Abstand, der zwischen der in proximale Richtung weisenden Anschlagfläche des Schaltnockens 17 und der in proximale Richtung weisenden Anschlagfläche herrscht, und dem Abstand der proximalen Begrenzungen der Aussparung 18 und des Sperrfensters 20.

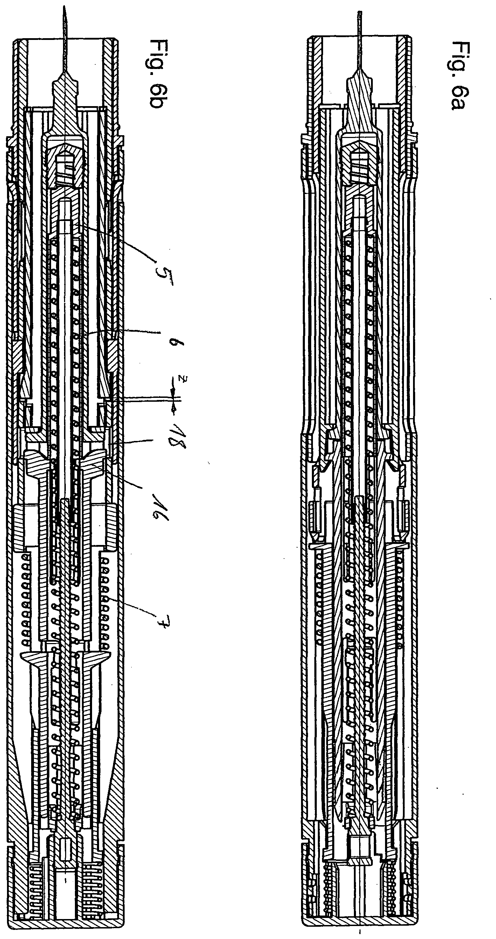

In den Figuren 6a und 6b wird die Injektionsvorrichtung in einem Zustand nach einer erfolgten Produktausschüttung gezeigt. Während der Produktausschüttung drückt die äußere Umfangsfläche des hülsenförmigen Teils der Kolbenstange 5 das Sperrelement 16 in die Aussparung 18, wodurch das Sperrelement 16 während einer Produktausschüttung gegen ein Ausrasten aus der Aussparung 18 gesichert ist. Die Kolbenstange 5 kann eine Aussparung aufweisen oder von der Länge her so bemessen sein, dass nach erfolgter Produktausschüttung die Sicherung des Sperrelements 16 durch die äußere Umfangsfläche der Kolbenstange 5 wegfällt, so dass das Sperrelement 16, wie in Figur 6b gezeigt wird, aus der Aussparung 18 ausrasten kann. Das Ausrasten kann aufgrund einer elastisch vorgespannten Anordnung des Sperrelements 16 oder aufgrund der Geometrie des Sperrelements 16, die ein Herausdrücken des Sperrelements 16 aus der Aussparung 18 verursacht, bewirkt werden.

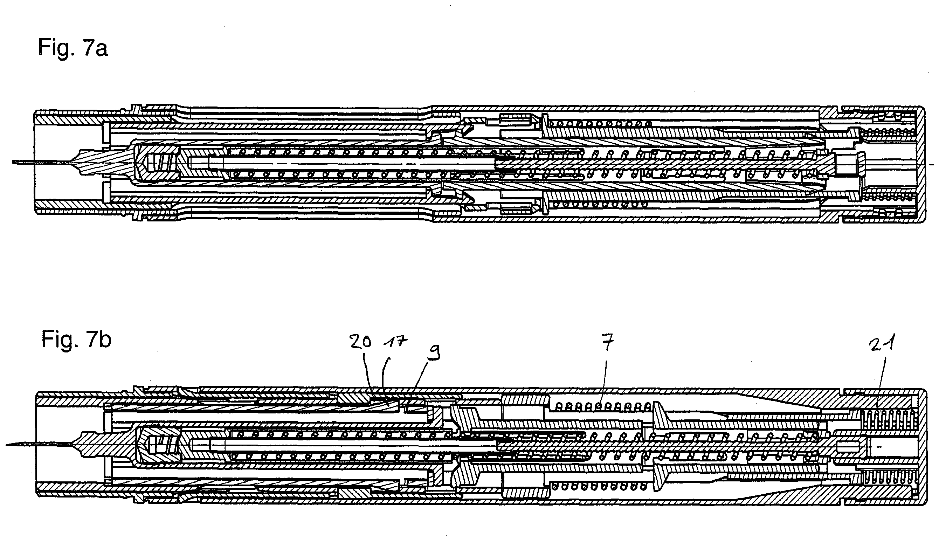

Am Ende der Produktausschüttung hat sich die Vortriebsfeder 6 weiter entspannt, während die Spannung der gespannten Rückzugsfeder 7 konstant geblieben ist. Die Federkraft der Vortriebsfeder 6 ist nun geringer, als die Federkraft der vorgespannten Rückzugsfeder 7. Durch das Lösen des Eingriffs des Sperrelements 16 mit der Aussparung 18 werden die Rückzugsfeder 7 und die Vortriebsfeder 6 wieder miteinander gekoppelt. Diese Kopplung bewirkt, dass wie in den Figuren 7a und 7b dargestellt ist, der kleine Abstand z (siehe Figuren 5b und 6b) verschwindet, indem die Vortriebsstruktur 2, 10, 11, d. h. insbesondere der Schaltnocken 17 mit seinem proximalen Ende schlagartig auf das distale Ende des Sperrfensters 20 bewegt wird. Beim Aufschlagen des Schaltnockens 17 wird ein haptisches und/oder ein akustisches Signal erzeugt. Durch diese Bewegung um den kleinen Weg z wird die Nadel 4 jedoch noch nicht vollständig aus dem Patienten herausgezogen. Der Patient bzw. der Verwender der Vorrichtung kann nun eine beliebige Zeit warten, bis die Nadel vollständig aus dem Patienten herausgezogen wird, da er den automatischen Nadelrückzug der Vorrichtung durch seinen Willen starten kann.

Eine vollständige Bewegung der Nadel in das distale Ende des Gehäuses 1 ist noch nicht möglich, da, wie aus Figur 7b ersichtlich ist, der Schaltnocken 17 in dem Eingriff mit dem Sperrfenster 20 ist und dadurch die Entspannung der Feder 7 blockiert. Um den Rückzug der Nadel 4 freizugeben, braucht der Verwender der Vorrichtung diese nur von der Injektionsstelle nehmen. Dadurch kann die Rückstellfeder 21 über die Schalthülse 8 die Betätigungshülse 9 in distale Richtung bewegen. Dabei steht die Vortriebsstruktur 2, 10, 11 relativ zur Betätigungshülse 9 fest, so dass der Schaltnocken 17 augrund seiner distalen Gestaltung angetrieben durch die Feder 21 in Verbindung mit der Betätigungshülse 9 mittels der Abragung 1d aus dem Sperrfenster 20 radial nach innen gedrückt wird. Sobald der Schaltnocken 17 nach innen gedrückt wurde, ist die Nadel 4 für den Rückzug frei. Außerdem wird durch das Lösen des Eingriffs die Rückzugsfeder 7 für eine Rückzugsbewegung freigegeben. Aufgrund der höheren Federkraft der vorgespannten Rückzugsfeder 7 wird die gesamte Vortriebsstruktur 2, 10, 11 in proximale Richtung gedrückt. Hierbei wird die Feder 6 wieder gespannt, wobei die Federkraft der Rückzugsfeder 7 während des gesamten Rückzugsvorgangs, d.h. auch bis zum Ende des Rückzugsvorgangs, größer ist als die Federkraft der Vortriebsfeder 6.

In den Figuren 9a und 9b wird die Injektionsvorrichtung in einem Endzustand gezeigt. In diesem Zustand hat die Injektionsvorrichtung wieder die gleichen Dimensionen wie am Anfang. Somit kann auch die Kappe 32 wieder aufgesetzt und die Injektionsvorrichtung entsorgt werden. In der Endposition ist die Nadel vollständig in das distale Ende der Vorrichtung eingezogen. Das Schnappelement 15 ist wieder mit der Schalthülse 8 wie am Anfang verrastet. Eine erneute Auslösung der Injektionsvorrichtung ist jedoch nicht möglich, da hierfür eine vorgespannte Vortriebsfeder 6, wie sie z. B. in Figur 1a gezeigt wird, notwendig wäre.

In den Figuren 10 und 11 wird die Signalisierungseinheit der Figuren 1 bis 9 im Detail gezeigt. Die Rasterstange 23 weist ein Raster 30 auf, welches eine Vielzahl von Rastelementen 31 umfasst, die entlang der Längsrichtung mit sich schrittweise verringernden Abständen angeordnet sind. Diese Abstände verringern sich anhand der nachlassenden Federkraft. Die Rasterstange ist mit seinem proximalen Ende, insbesondere mit seinem Kopf zumindest in eine Richtung axial fest mit der Schalthülse 8 (z. B. Figur 1) verbunden. Die Rasterstange 23 ist von einer Rasterhülse 22 umgeben, die mit dem distalen Ende der Vortriebsfeder 6 oder/und mit dem distalen Endbereich der Kolbenstange 5 verbunden ist. Die Eingriffshülse weist ein Eingriffselement 26 auf, das in eine ringförmige Nut 27 eingreift. Insbesondere greift das Eingriffselement 26 in der Ausgangsposition in die Nut 27 ein. Bei der Vorschubbewegung zum Einstechen, d.h. bei der Einstechbewegung rastet das Eingriffselement aus der Nut 27 aus und bewegt sich über einen ersten Abschnitt der Rasterstange bis zum Anfang der Vielzahl der Rastelemente 31. Der erste Abschnitt weist kein weiteres Rastelement auf sondern ist im Wesentlichen zylinderförmig oder sich verjüngend, so dass bei der Einstechbewegung keine Signale abgegeben werden. Grundsätzlich sind Ausführungsformen, bei denen dies möglich ist vorteilhaft. Die Länge des ersten Abschnitts ist so bemessen, dass das Eingriffselement 26 im Wesentlichen den ersten Abschnitt vollständig durchfahren hat bei Beendigung der Einstichbewegung. Beim Start der Ausschüttbewegung werden Stange 23 und Hülse 22 noch weiter auseinander gezogen, so dass das Eingriffselement 26 sich über den zweiten Abschnitt, d.h. den Abschnitt mit dem Rastelement 31, bewegt, so dass die Rastelemente 31 jeweils überfahren werden. Bei jedem Überfahren der Rastelemente wird ein kurzes Klick-Signal abgegeben. Die Zeitintervalle von einem Klick-Signal bis zum nächsten sind konstant, obwohl die nachlassende Federkraft die Geschwindigkeit des Eingriffselements 26 mit zunehmendem Weg verringert. Erfindungsgemäß verringern sich die Abstände von einem Rastelement zum nächsten mit zunehmendem Federweg. Dadurch wird der sich verändernden Geschwindigkeit Rechnung getragen.

Auf der radial gegenüberliegenden Seite, an der das Eingriffsglied 26 angeordnet ist, kann beispielsweise ein weiteres Eingriffsglied 26 vorgesehen sein. Bevorzugt ist, wie hier dargestellt wird, kein weiteres Eingriffsglied 26 vorgesehen, sondern lediglich eine von der Hülsenwandung gebildete Stütze, die als Widerlager dient.

In den Figuren 12 bis 14 wird eine alternative Ausführungsform der Signalisierungseinheit für die Injektionsvorrichtung aus den Figuren 1 bis 9 gezeigt. Das Raster 30 ist in einer Nut 29, nämlich an dessen Flanke angeordnet. Die Rastelemente 31 stehen in Umfangsrichtung von der Nutflanke ab. In der Nut 29 ist ein axial bewegbarer Schlitten 28 angeordnet, der axial fest mit der Kolbenstange 5 gekoppelt ist. Bei der Ausschüttbewegung wird der Schlitten 28 durch die Kolbenstange 5 mitgenommen, wodurch das am Schlitten 28 federnd angeordnete Eingriffsglied 26 die einzelnen Rastelemente 31 des Rasters 30 überfährt. Auch hier haben die Rastelemente 31 jeweils Abstände voneinander, die der sich verändernden Kraft der Vortriebsfeder für eine zeitkonstante Abgabe von Signalen Rechnung tragen. Der Abstand der Sägezähne ist also gewählt, dass die einzelnen Klicks in gleichmäßigen Zeitabständen erfolgen, obwohl der Schlitten 28 mit der Kolbenstange 5 am Ende der Ausschüttung eine geringere Ausschüttgeschwindigkeit ausweist als am Anfang.

Figuren 15 und 16 zeigen eine weitere Ausführungsform der Signalisierungseinheit, bei der das Raster 30 aus Ausnehmungen, insbesondere Fenstern gebildet ist, die ebenfalls mit sich verändernden Abständen auf der Kolbenstange 5 angebracht sind. Das Eingriffsglied 26 ist an der Funktionshülse 11 federnd angeordnet. Bei der Ausschüttbewegung wird die Kolbenstange 5 und somit das Lochraster 30 an dem Eingriffsglied 26 vorbeibewegt, das jeweils in jedes Lochraster 31 einrastet und somit das Signal erzeugt. Der Vorteil an dieser Ausführungsform ist, dass das Eingriffsglied 26 von dem Sperrelement 16 gebildet werden kann, so dass diese Ausfuhrungsform mit besonders wenigen Teilen auskommt.

### Bezugszeichenliste

- 1: Gehäuse
- 1a: proximales Gehäuseteil
- 1b: distales Gehäuseteil
- 1c: Rastverbindung
- 1d: Abragung
- 2: Produktbehältnis
- 3: Kolben
- 4: Injektionsnadel
- 5: Kolbenstange
- 6: Vortriebsfeder
- 7: Rückzugsfeder
- 8: Schalthülse
- 8a: Sockel
- 9: Betätigungshülse
- 10: Produktbehältnishalterung
- 11: Funktionshülse
- 11a: Bund
- 12: Sichtfenster
- 13: Aktivierungselement
- 13a: Aktivierungsnocken
- 14: Aktivierungssicherung
- 15: Schnappelement
- 16: Sperrelement
- 17: Schaltnocken
- 18: Aussparung
- 19: Axialanschlag
- 20: Sperrfenster
- 21: Rückstellfeder
- 22: Eingriffshülse
- 23: Rasterstange
- 24: Kopf
- 25: Gleitführung
- 26: Eingriffsglied
- 27: Nut
- 28: Schlitten
- 29: Nut
- 30: Raster
- 31: Rastelement
- 32: Kappe

- z: Abstand
- x₀: Abstand
- x₀-Δx: veränderter Abstand
- L: Längsachse

## Patentansprüche

1. Injektionsvorrichtung, insbesondere Autoinjektor, umfassend:
a) ein Gehäuse (1),
b) eine Betätigungshülse (9), die relativ zu dem Gehäuse (1) bewegbar gelagert und mit dem distalen Ende der Injektionsvorrichtung auf eine Injektionsstelle eines Patienten aufsetzbar ist,
c) eine Injektionsnadel (4), welche durch einen Mechanismus der Injektionsvorrichtung in eine Einstechposition bewegt wird, in der Einstechposition über das distale Ende der Injektionsvorrichtung hervorsteht und ins Innere der Injektionsvorrichtung zurückbewegbar ist,
**gekennzeichnet durch**
d) einen Schaltnocken (17), der axial fest zu der Injektionsnadel (4) angeordnet ist und in der Einstechposition in ein Sperrfenster (20) eingreift, um eine Rückzugsbewegung der Injektionsnadel (4) in das Innere der Injektionsvorrichtung zu verhindern,
e) wobei durch eine Bewegung der Betätigungshülse (9) in distale Richtung eine in Längsrichtung (L) der Injektionsvorrichtung weisende Breite des Sperrfensters (20) so verringert wird, dass der Schaltnocken (17) aus dem Eingriff mit dem Sperrfenster (20) bewegt wird, so dass die Injektionsnadel (4) für die Rückzugsbewegung in das Innere der Injektionsvorrichtung freigegeben ist.

2. Injektionsvorrichtung nach dem vorhergehenden Anspruch, wobei das Gehäuse (1) oder ein gehäusefestes Element das Sperrfenster (20) distal und die Betätigungshülse (9) das Sperrfenster (20) proximal begrenzen.

3. Injektionsvorrichtung nach einem der vorhergehenden Ansprüche, wobei die in Längsrichtung (L) der Injektionsvorrichtung weisende Breite des Sperrfensters (20) veränderbar ist, so dass je nach Breite des Sperrfensters (20) der Schaltnocken (17) in den oder aus dem Eingriff mit dem Sperrfenster (20) bewegbar ist.

4. Injektionsvorrichtung nach einem der vorhergehenden Ansprüche, wobei der Schaltnocken (17) eine in proximale Richtung weisende Anschlagfläche aufweist, die mit der Betätigungshülse (9) so zusammenwirken kann, dass der Schaltnocken (17) von der Betätigungshülse (9) bei einer Bewegung der Betätigungshülse in distale Richtung mitgenommen werden kann, wenn sich der Schaltnocken (17) in dem Sperrfenster (20) befindet.

5. Injektionsvorrichtung nach einem der vorhergehenden Ansprüche, wobei der Schaltnocken (17) in distaler Richtung so geformt ist, dass er von dem Gehäuse (1) oder dem gehäusefesten Element aus dem Eingriff mit dem Sperrfenster (20) bewegbar ist oder/und dass er von der Betätigungshülse (9) aus dem Eingriff mit dem Sperrfenster (20) mit einer von der Betätigungshülse (9) gebildeten Aussparung bewegbar ist.

6. Injektionsvorrichtung nach einem der vorhergehenden Ansprüche, ferner umfassend ein Elastizitätsmittel (21), insbesondere Feder, welches durch eine Bewegung der Betätigungshülse (9) in proximale Richtung spannbar ist und mit dem die Betätigungshülse (9) für eine Bewegung in distale Richtung antreibbar ist.

7. Injektionsvorrichtung nach einem der vorhergehenden Ansprüche, wobei sich der Schaltnocken (17) bei einer in das Innere der Injektionsvorrichtung zurückgezogenen Position, insbesondere in einer Ausgangsposition und/oder einer Endposition der Injektionsnadel (4), in einem Eingriff mit einer von der Betätigungshülse (9) proximal des Sperrfensters (20) gebildeten Aussparung (18) befindet.

8. Injektionsvorrichtung nach einem der vorhergehenden Ansprüche, wobei das Gehäuse (1) oder ein gehäusefestes Element eine radial nach innen weisende Abragung (1d) aufweist, die in einen von der Betätigungshülse (9) gebildeten Durchbruch soweit eingreift, dass die radial nach innen weisende Innenfläche der Abragung (1d) in etwa bündig mit der radial nach innen weisenden Innenfläche der Betätigungshülse (9) ist.

9. Injektionsvorrichtung nach einem der vorhergehenden Ansprüche, wobei der Schaltnocken (17) federnd an einem Teil einer Vortriebsstruktur (2,10,11), insbesondere an einer Halterung (10) für ein Produktbehältnis (2), gebildet ist.

10. Injektionsvorrichtung nach einem der vorhergehenden Ansprüche, wobei der Schaltnocken (17) mit einer Rückzugfeder (7) gekoppelt ist, mit der der Schaltnocken (17) und die damit axial fest verbundene Injektionsnadel (4) in proximale Richtung bewegbar sind.

11. Injektionsvorrichtung nach einem der vorhergehenden Ansprüche, ferner umfassend ein Sperrelement, (16) welches mit dem Schaltnocken (17) über eine Vortriebsstruktur (2, 10, 11) axial fest verbunden ist und welches in der Einstechposition der Injektionsnadel (4) in einen Eingriff mit einer proximal des Sperrfensters (20) von der Betätigungshülse (9) gebildeten Aussparung (18) bewegbar ist.

12. Injektionsvorrichtung nach Anspruch 11, wobei in der Einstechposition der Nadel (4) der Schaltnocken (17) sich in einem Eingriff mit dem Sperrfenster (20) und das Sperrelement (16) sich in einem Eingriff mit der Aussparung (18) befindet.

13. Injektionsvorrichtung nach Anspruch 4 und 12, wobei sich zwischen einer proximalen Begrenzung des Sperrfensters (20) und der in proximale Richtung weisenden Anschlagfläche des Schaltnockens (17) ein kleiner Abstand (Z), insbesondere von 0,3 bis 3mm, befindet, wobei das Sperrelement (16) mit seiner in proximale Richtung weisenden Anschlagfläche in Berührung mit einer proximalen Begrenzung der Aussparung (18) der Betätigungshülse (9) ist.

14. Injektionsvorrichtung nach dem vorhergehenden Anspruch und den Ansprüchen 9 und 10, wobei die Vortriebsstruktur (2, 10, 11) von der Rückzugfeder (7) um den Betrag bewegbar ist, der dem kleinen Abstand (Z) entspricht, wodurch ein haptisches, insbesondere taktiles, oder akustisches Signal erzeugt wird.

## Claims

1. An injection device, in particular an autoinjector, including:
a) a housing (1),
b) an actuating sleeve (9) which is mounted moveably relative to the housing (1) and which can be placed with the distal end of the injection device on an injection site of a patient,
c) an injection needle (4) which is moved into an insertion position by a mechanism of the injection device, projects in the insertion position beyond the distal end of the injection device and can be moved back into the interior of the injection device,
**characterised by**
d) a switching cam (17) which is arranged axially fixed with respect to the injection needle (4) and in the insertion position engages into a locking window (20) to prevent a return movement of the injection needle (4) into the interior of the injection device,
e) wherein a width of the locking window (20) facing in the longitudinal direction (L) of the injection device is reduced by a movement of the actuating sleeve (9) in the distal direction so that the switching cam (17) is moved out of the engagement with the locking window (20) so that the injection needle (4) is released for the return movement into the interior of the injection device.

2. An injection device according to the preceding claim wherein the housing (1) or an element fixed with respect to the housing delimits the locking window (20) distally and the actuating sleeve (9) delimits the locking window (20) proximally.

3. An injection device according to one of the preceding claims wherein the width of the locking window (20) facing in the longitudinal direction (L) of the injection device is variable so that depending on the respective width of the locking window (20) the switching cam (17) is moveable into or out of engagement with the locking window (20).

4. An injection device according to one of the preceding claims wherein the switching cam (17) has an abutment surface which faces in the proximal direction and which can cooperate with the actuating sleeve (9) in such a way that the switching cam (17) can be entrained by the actuating sleeve (9) upon a movement of the actuating sleeve in the distal direction when the switching cam (17) is in the locking window (20).

5. An injection device according to one of the preceding claims wherein the switching cam (17) is so shaped in the distal direction that it is moveable by the housing (1) or the element fixed with respect to the housing out of engagement with the locking window (20) and/or that it is moveable by the actuating sleeve (9) out of engagement with the locking window (20) with an aperture formed by the actuating sleeve (9).

6. An injection device according to one of the preceding claims and further including an elasticity means (21), in particular a spring, which can be stressed by a movement of the actuating sleeve (9) in the proximal direction and with which the actuating sleeve (9) is drivable for a movement in the distal direction.

7. An injection device according to one of the preceding claims wherein in a position of being retracted into the interior of the injection device, in particular in a starting position and/or an end position of the injection needle (4), the switching cam (17) is in engagement with an aperture (18) formed by the actuating sleeve (9) proximally of the locking window (20).

8. An injection device according to one of the preceding claims wherein the housing (1) or an element fixed with respect to the housing has a radially inwardly facing projection (1d) which engages into an opening formed by the actuating sleeve (9) to such an extent that the radially inwardly facing inside surface of the projection (1d) is approximately flush with the radially inwardly facing inside surface of the actuating sleeve (9).

9. An injection device according to one of the preceding claims wherein the switching cam (17) is formed resiliently on a part of a drive structure (2, 10, 11), in particular on a holder (10) for a product container (2).

10. An injection device according to one of the preceding claims wherein the switching cam (17) is coupled to a return spring (7), with which the switching cam (17) and the injection needle (4) axially fixedly connected thereto are moveable in the proximal direction.

11. An injection device according to one of the preceding claims and further including a locking element (16) which is axially fixedly connected to the switching cam (17) by way of a drive structure (2, 10, 11) and which in the insertion position of the injection needle (4) is moveable into engagement with an aperture (18) formed by the actuating sleeve (9) proximally of the locking window (20).

12. An injection device according to claim 11 wherein in the insertion position of the needle (4) the switching cam (17) is in engagement with the locking window (20) and the locking element (16) is in engagement with the aperture (18).

13. An injection device according to claim 4 and claim 12 wherein there is a small spacing (Z), in particular of 0.3 to 3 mm, between a proximal boundary of the locking window (20) and the abutment surface facing in the proximal direction of the switching cam (17), wherein the locking element (16) with its abutment surface facing in the proximal direction is in contact with a proximal boundary of the aperture (18) of the actuating sleeve (9).

14. An injection device according to the preceding claim and claims 9 and 10 wherein the drive structure (2, 10, 11) is moveable by the return spring (7) by the amount which corresponds to the small spacing (Z), whereby a haptic, in particular tactile, or acoustic signal is produced.

## Revendications

1. Dispositif d'injection, en particulier auto-injecteur, comprenant :
a) un boîtier (1),
b) une douille d'actionnement (9) qui est logée mobile par rapport au boîtier (1) et peut être placée avec l'extrémité distale du dispositif d'injection sur un point d'injection d'un patient,
c) une aiguille d'injection (4), qui est déplacée dans une position de piqûre par un mécanisme du dispositif d'injection, fait saillie de l'extrémité distale du dispositif d'injection dans la position de piqûre et peut être ramenée à l'intérieur du dispositif d'injection,
**caractérisé par**
d) une came de commutation (17), qui est agencée de manière axialement fixe par rapport à l'aiguille d'injection (4) et vient en prise avec une fenêtre de blocage (20) dans la position de piqûre pour empêcher un mouvement de retrait de l'aiguille d'injection (4) vers l'intérieur du dispositif d'injection,
e) dans lequel par un mouvement de la douille d'actionnement (9) dans la direction distale, une largeur de la fenêtre de blocage (20) tournée dans la direction longitudinale (L) du dispositif d'injection est réduite de sorte que la came de commutation (17) soit séparée de la fenêtre de blocage (20), de sorte que l'aiguille d'injection (4) soit libérée pour le mouvement de retrait vers l'intérieur du dispositif d'injection.

2. Dispositif d'injection selon la revendication précédente, dans lequel le boîtier (1) ou un élément fixé au boîtier délimite la fenêtre de blocage (20) de manière distale et la douille d'actionnement (9) délimite la fenêtre de blocage (20) de manière proximale.

3. Dispositif d'injection selon l'une quelconque des revendications précédentes, dans lequel la largeur de la fenêtre de blocage (20) tournée dans la direction longitudinale (L) du dispositif d'injection est modifiable de sorte qu'en fonction de la largeur de la fenêtre de blocage (20), la came de commutation (17) puisse être mise en prise avec la fenêtre de blocage (20) ou séparée de celle-ci.

4. Dispositif d'injection selon l'une quelconque des revendications précédentes, dans lequel la came de commutation (17) présente une surface de butée tournée dans la direction proximale, qui peut coopérer avec la douille d'actionnement (9) de telle sorte que la came de commutation (17) peut être entraînée par la douille d'actionnement (9) lors d'un mouvement de la douille d'actionnement dans la direction distale, lorsque la came de commutation (17) se trouve dans la fenêtre de blocage (20).

5. Dispositif d'injection selon l'une quelconque des revendications précédentes, dans lequel la came de commutation (17) est formée dans la direction distale de telle sorte qu'elle peut être séparée, par le boîtier (1) ou l'élément fixé au boîtier, de la fenêtre de blocage (20) ou/et qu'elle peut être séparée, par la douille d'actionnement (9), de la fenêtre de blocage (20) avec un évidement formé par la douille d'actionnement (9).

6. Dispositif d'injection selon l'une quelconque des revendications précédentes, comprenant en outre un moyen d'élasticité (21), en particulier ressort, qui peut être tendu par un mouvement de la douille d'actionnement (9) dans la direction proximale et avec lequel la douille d'actionnement (9) peut être entraînée pour un mouvement dans la direction distale.

7. Dispositif d'injection selon l'une quelconque des revendications précédentes, dans lequel la came de commutation (17) se trouve, dans une position retirée à l'intérieur du dispositif d'injection, en particulier dans une position initiale et/ou une position finale de l'aiguille d'injection (4), en prise avec un évidement (18) formé par la douille d'actionnement (9) de manière proximale à la fenêtre de blocage (20).

8. Dispositif d'injection selon l'une quelconque des revendications précédentes, dans lequel le boîtier (1) ou un élément fixé au boîtier présente une saillie (1d) tournée radialement vers l'intérieur, qui vient en prise avec un passage formé par la douille d'actionnement (9), de sorte que la surface intérieure de la saillie (1d) tournée radialement vers l'intérieur soit à peu près à fleur avec la surface intérieure de la douille d'actionnement (9) tournée radialement vers l'intérieur.

9. Dispositif d'injection selon l'une quelconque des revendications précédentes, dans lequel la came de commutation (17) est formée de manière élastique au niveau d'une partie d'une structure d'avancement (2, 10, 11), en particulier au niveau d'une fixation (10) pour un récipient de produit (2).

10. Dispositif d'injection selon l'une quelconque des revendications précédentes, dans lequel la came de commutation (17) est couplée à un ressort de retrait (7), avec lequel la came de commutation (17) et l'aiguille d'injection (4) qui y est fixée axialement sont mobiles dans la direction proximale.

11. Dispositif d'injection selon l'une quelconque des revendications précédentes, comprenant en outre un élément de blocage (16), qui est fixé axialement à la came de commutation (17) via une structure d'avancement (2, 10, 11) et qui, dans la position de piqûre de l'aiguille d'injection (4), peut être mis en prise avec un évidement (18) formé par la douille d'actionnement (9) de manière proximale à la fenêtre de blocage (20).

12. Dispositif d'injection selon la revendication 11, dans lequel dans la position de piqûre de l'aiguille (4), la came de commutation (17) est en prise avec la fenêtre de blocage (20) et la fenêtre de blocage (16) est en prise avec l'évidement (18).

13. Dispositif d'injection selon la revendication 4 et 12, dans lequel une petite distance (Z), en particulier de 0,3 à 3 mm, se trouve entre une délimitation proximale de la fenêtre de blocage (20) et la surface de butée de la came de commutation (17) tournée dans la direction proximale, dans lequel l'élément de blocage (16) est en contact avec sa surface de butée tournée dans la direction proximale avec une délimitation proximale de l'évidement (18) de la douille d'actionnement (9).

14. Dispositif d'injection selon la revendication précédente et les revendications 9 et 10, dans lequel la structure d'avancement (2, 10, 11) peut être déplacée par le ressort de retrait (7) de la valeur qui correspond à la petite distance (Z), ce par quoi un signal haptique, en particulier tactile ou acoustique, est généré.
